# EUROPEAN PATENT SPECIFICATION

(11) **EP 2 654 776 B1**
(45) Date of publication and mention of the grant of the patent: **21.11.2018**
(21) Application number: 11850102.2
(22) Date of filing: 16.12.2011
(51) Int. Cl.: A61K 38/50, A61K 47/60, A61P 35/02

(54) **USE OF PEGYLATED RECOMBINANT HUMAN ARGINASE FOR TREATMENT OF LEUKEMIA**
VERWENDUNG VON PEGYLIERTER REKOMBINANTER HUMANER ARGINASE ZUR BEHANDLUNG VON LEUKÄMIE
UTILISATION D'ARGINASE HUMAINE RECOMBINANTE PÉGYLÉE POUR LE TRAITEMENT D'UNE LEUCÉMIE

(30) Priority: 21.12.2010 US 201061425243 P
(43) Date of publication of application: 30.10.2013
(73) Proprietor: Bio-Cancer Treatment International Ltd., Hong Kong Science Park, Sha Tin Hong Kong (CN)
(72) Inventor: CHENG, Ning Man, Central, Hong Kong (CN)
(74) Representative: Cabinet Chaillot
(86) International application number: PCT/IB2011/055735
(87) International publication number: WO 2012/085793

(56) References cited:
- WO-A1-2004/000349
- WO-A1-2006/058486
- WO-A2-2012/061015
- C. P. HERNANDEZ ET AL: "Pegylated arginase I: a potential therapeutic approach in T-ALL", BLOOD, vol. 115, no. 25, 20 April 2010 (2010-04-20), pages 5214-5221, XP055118284, ISSN: 0006-4971, DOI: 10.1182/blood-2009-12-258822
- SAVOCA K V ET AL: "CANCER THERAPY WITH CHEMICALLY MODIFIED ENZYMES. II. THE THERAPEUTIC EFFECTIVENESS OF ARGINASE AND ARGINASE MODIFIED BY THE COVALENT ATTACHMENT OF POLYETHYLENE GLYCOL ON THE TAPER LIVER TUMOR AND THE L5178Y MURINE LEUKEMIA", CANCER BIOCHEMISTRY BIOPHYSICS, GORDON AND BREACH SCIENCE PUBLISHER, INC, US, vol. 7, no. 3, 1 January 1994 (1994-01-01) , pages 261-268, XP008007608, ISSN: 0305-7232
- HERMANDEZ, CLAUDIA P. ET AL.: 'Pegylated arginase I: a potential therapeutic approach in T-ALL.' BLOOD. vol. 115, no. 25, 24 June 2010, pages 5214 - 5221, XP055118284

## Description

### CROSS-REFERENCE TO RELATED APPLICATION

This application claims benefit under 35 U.S.C. § 119(e) of U.S. Provisional Application having Serial No. 61/425,243 filed on Dec. 21, 2010.

### FIELD OF INVENTION

This invention relates to method of treating leukemia with arginase. In particular, the method relates to treatment of leukemia with pegylated recombinant human arginase.

### BACKGROUND OF INVENTION

Haematologic malignancies, such as non-Hodgkin's lymphoma and leukemia, rank number 10 in the most common cancers worldwide. Acute lymphocytic leukemia is one of the most common pediatric malignances and remains the leading cause of death from a disease in children despite the high curing rates achieved with contemporary regimens. In adults, hemic malignanices account for about 10% of all cancers. Chemotherapy together with target therapy remains the mainstay of treatment. On relapse, bone marrow transplantation offers the only means of cure. However, this modality of treatment can only be offered to patients with suitable Human leukocyte antigen (HLA) compatible donors. For the unfortunate patients with refractory leukemias and lymphomas without suitable marrow donors, prognosis is grim.

The standard of care for leukemia and lymphoma is chemotherapy given systemically and intrathecally in conjunction with various target therapies such as rituximab, anti-CD30, Campath etc. Often, radiation is employed for cranial prophylaxis and local therapy for lymphomas, in particular the Hodgkin's lymphomas. In patients with relapsed leukemia and lymphoma, infusion of HLA compatible stem cells either from related donors or unrelated donors following high-dose chemotherapy as in the case of bone marrow transplantation can be curative, but with high morbidity and a modest treatment-related death. For those patients with refractory disease in absence of suitable HLA donors, no standard treatment exists. Patients can be considered for clinical trials or given palliation; in either case, prognosis is extremely poor. Therefore there is clearly a need for a new and improved treatment method.

Savoca, K.V. et. al., (1984) Cancer Biochem Biophys, Vol. 7, pp. 261-268 discloses suppressive effects of PEG-arginase on L5178Y leukemia cells in vitro. The document is silent on the use of arginase in treating leukemia in a patient, wherein said lymphocytic and/or myeloid leukemia is arsenic resistant.

### SUMMARY OF INVENTION

The present invention, as set out in the claims, provides a composition comprising arginase for use in a method for treating leukemia in a patient, wherein said lymphocytic and/or myeloid leukemia is arsenic resistant.

Any subject matter falling outside the scope of the claims is provided for information only. Any references in the description to methods of treatment refer to the compounds, pharmaceutical compositions and medicaments of the present invention for use in a method for treatment of the human or animal body by therapy. The method involves the administration to the patient a therapeutically effective amount of a composition comprising arginase, wherein said composition is effective at treating lymphocytic and/or myeloid leukemia.

In an exemplary embodiment of the present invention, the lymphocytic leukemia is acute. In another exemplary embodiment, the lymphocytic leukemia is chronic.

In another exemplary embodiment of the present invention, the lymphotic leukemia is T-cell acute lymphocytic leukemia.

In an exemplary embodiment of the present invention, the myeloid leukemia is acute. In another exemplary embodiment, the myeloid leukemia is chronic.

In yet another exemplary embodiment of the present invention, the myeloid leukemia is arsenic resistant myeloid leukemia.

In another exemplary embodiment, the arginase is pegylated recombinant human arginase. In a further embodiment, the pegylating agent is methoxy poly(ethylene glycol) succinimidyl propionate (mPEG-SPA).

In a further aspect of the present invention, a method of treating arsenic resistant leukemia is provided. In this method a therapeutically effective amount of a composition comprising arginase is administrated to a patient, wherein the composition is effective at treating arsenic resistant lymphocytic and/or arsenic resistant myeloid leukemia. In an exemplary embodiment, the arginase is pegylated recombinant human arginase. In a further exemplary embodiment, the pegylating agent is methoxy poly(ethylene glycol) succinimidyl propionate (mPEG-SPA).

The arginase of the present invention can be administrated in combination with a second therapeutic agent. In an exemplary embodiment, the second therapeutic agent is Doxorubicin.

In yet a further aspect, the arginase comprising composition of the present invention is administrated intravenously, intraperitoneally, subcutaneously, or intramuscularly.

### BRIEF DESCRIPTION OF FIGURES

Fig. 1a and Fig. 1b show respectively the effect of arsenic trioxide (As₂O₃) and BCT-100 on cell viability in various promyelocytic leukemia cell lines.
Fig. 2 shows the effect of BCT-100 and arsenic trioxide in inducing apoptosis in various promyelocytic leukemia cell lines.
Fig. 3 shows BCT-100 induces apoptosis via inhibition of pmTOR and induction of Stat3 and Bax.
Fig. 4a shows the induction of granulocytic differentiation in NB4 by BCT-100. Fig. 4b shows the BCT-100 induced differentiation in NB4 and HL60 cells. Fig 4c shows the granulocytic morphology of BCT-100 treated NB4 cells.
Fig. 5a and Fig 5b show the reorganization of promyelocytic leukemia nuclear bodies in HL60 cells after BCT-100 treatment
Fig. 6 shows the IC₅₀ of BCT-100 in various cancer cell lines.
Fig. 7 shows the effect of BCT-100 on various leukemia cell lines when administrated in combination with Doxorubicin.
Fig. 8 shows the effect of BCT-100 on the liver, spleen and sternum cytology of HL60-incoluated NOD/SCIDS mice.
Fig. 9 shows the enhanced survival rate of HL60-incoluated NOD/SCIDS mice when treated with BCT-100.

### DETAILED DESCRIPTION OF THE PREFERRED EMBODIMENTS

As used herein and in the claims, "comprising" means including the following elements but not excluding others.

The present invention provides the use of arginase in treating leukemia. In certain embodiments, pegylated recombinant human arginase is used for the treatment of various types of leukemia. In a further embodiment, the pegylated recombinant human arginase is BCT-100 and the preparation of pegylated recombinant human arginase and the steps for pegylating the same are disclosed in, e.g., US 10/518,223.

Arginase is a manganese-containing enzyme, catalyzing the conversion of arginine to ornithine and urea, the last step of the Urea Cycle. Pegylated recombinant human arginase in our preclinical study showed efficacious in inducing arginine depletion in dose-dependent manner.

The present invention also provides the use of arginase, for example, pegylated recombinant human arginase such as BCT-100 in treating cancer in a patient who suffers from refractory or relapsed leukemia or lymphoma. The term "refractory or relapsed leukemia or lymphoma" refers to the condition of a patent suffering from leukemia or lymphoma that the patient is unresponsive to all available drugs for treating leukemia or lymphoma, or signs and symptoms return to the patients upon using all other available drugs for treating leukemia or lymphoma.

The present invention shows that arginase, for example, pegylated recombinant human arginase such as BCT-100 is at least 6-10 times more effective in treating T-cell leukemia and myeloid leukemia than treating hepatocellular carcinoma.

The arginase of the present invention is shown to be effective in treating various types of leukemia, e.g., lymphocytic leukemia including but not limited to T-cell leukemia; myeloid leukemia including but not limited to promyelocytic leukemia.

The arginase of the present invention can be administrated in combination with a second therapeutic agent such as Doxorubicin. In various embodiments, enhanced therapeutic effect was observed when BCT-100 was administrated in combination with Doxorubicin.

The present invention also shows that arginase, for example, pegylated recombinant human arginase such as BCT-100 is effective in treating both arsenic sensitive and arsenic resistant leukemia. The effectiveness of BCT-100 in inducing apoptosis in both arsenic sensitive and arsenic resistant leukemia is also demonstrated in the present invention.

The present invention is further defined by the following examples.

### Example 1

### The Inhibitory Effect Of BCT-100 On Various Leukemia Cell Lines

The inhibitory effect of BCT-100 in acute myeloid leukemia cells (Kasumi-1a, ML2, HL60, K562 and NB4) and T-cell leukemia cells (Jurkat, ALL-SIL, HPB-ALL and TALL-1) was studied. The IC₅₀ values of BCT-100 in various cell lines are shown in Table 1. The result indicates that BCT-100 is effective in inhibiting the growth of leukemia, including myeloid leukemia and lymphocytic leukemic.

**Table 1 - IC₅₀ of BCT-100 in various leukemia cell lines.**

| Myeloid Leukemia | IC₅₀ (mU/mL) | T-cell Leukemia | IC₅₀ (mU/mL) |
|---|---|---|---|
| Kasumi-1a | 65 | Jurkat | 40 |
| ML2 | 65 | All-SIL | 120 |
| HL60 | 55 | HPB-ALL | 110 |
| K562 | 25 | TALL-1 | 100 |
| NB4 | 60 | | |

### Example 2

### Effect of BCT-100 On Arsenic Sensitive and Arsenic Resistant Myelocytic Cell Lines

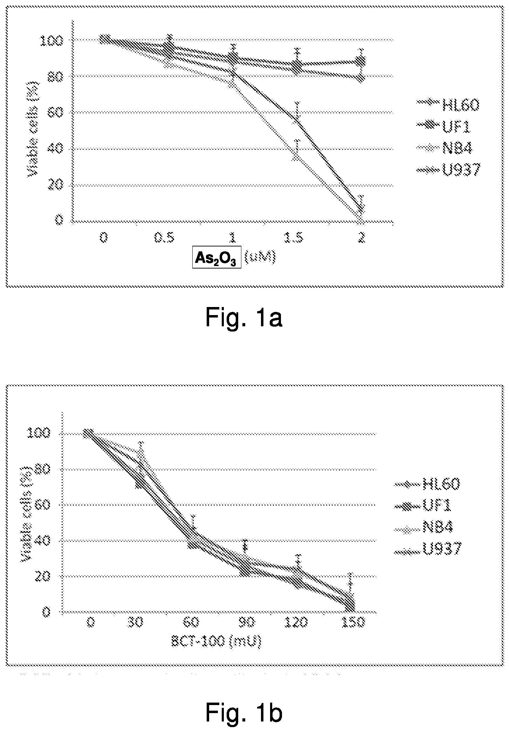

The effect of BCT-100 on arsenic sensitive myelocytic cell lines (NB4 and U937) as well as arsenic resistant myelocytic cell lines (HL60 and UF1) was investigated. Fig. 1a shows the effect of arsenic trioxide on cell viability in myelocytic leukemia cells. The cell viability in the arsenic sensitive NB4 and U937 cell lines dropped as the concentration of arsenic increased. However, HL60 and UF1 cell lines did not respond to As₂O₃ treatment.

Fig. 1b shows the effect of BCT-100 on cell viability in myelocytic leukemia cells NB4, U937, HL60 and UF1. Both the arsenic sensitive and resistant leukemia cells responded to the BCT-100 treatment. In all cases the cell viability dropped with increasing amount of BCT-100 in the medium.

The results showed BCT-100 is effective in inhibiting the growth of myelocytic leukemia, including myelocytic leukemia that is resistant to arsenic. Therefore, the arginase of the present invention is useful for treating arsenic resistant leukemia.

### Example 3

### Effect of BCT-100 In Inducing Apoptosis In Leukemia Cells

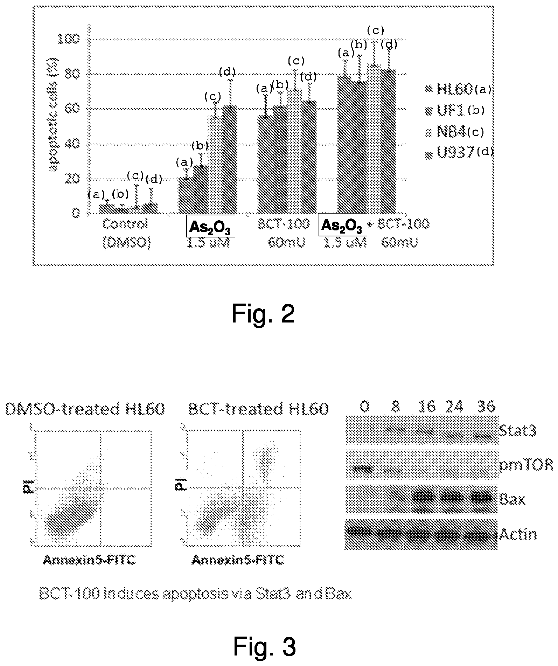

The effect of BCT-100 in inducing apoptosis in both arsenic sensitive (NB4 and U937) and arsenic resistant (HL60 and UF1) leukemia cell lines was tested. As shown in Fig. 2, arsenic was effective in inducing apoptosis in leukemic cell lines NB4 and U937.However, the apoptosis rate was low in HL60 and UF1 cells in the presence of arsenic, as these cell lines are arsenic resistant. BCT-100 was found to be effective in inducing apoptosis in both arsenic sensitive and arsenic resistant leukemia cell lines. The apoptosis rate was further enhanced when BCT-100 is administrated in combination with arsenic trioxide.

### Example 4

### Mechanism Of BCT-100 Induced Apoptosis

Expression of Bax and pmTOR was assessed in leukemic cell line HL60 by western blotting with or without BCT-100 treatment using monoclonal antibodies against Bax and pmTOR respectively. The same blot was stripped and probed with anti-actin antibody for loading control. Apoptosis was determined by annexin V labeling. HL60 cells were cultured in medium with or without BCT-100 and the expression of annexin V were analyzed by flow-cytometry at 8, 16, 24, and 36 hrs after BCT-100 treatment.

As revealed by western blot analysis shown in Fig. 3, the Bax level was up-regulated 8 hrs after BCT-100 treatment and remained high levels afterwards. The protein level of pmTOR was down-regulated in response to BCT-100 treatment. These results indicate that BCT-100 may induce apoptosis of leukemic cell line HL60 through signal transduction pathway involving Bax/Bcl-2 or specifically due to inhibition of pmTOR signaling.

### Example 5

### Induction Of Granulocytic Differentiation By BCT-100

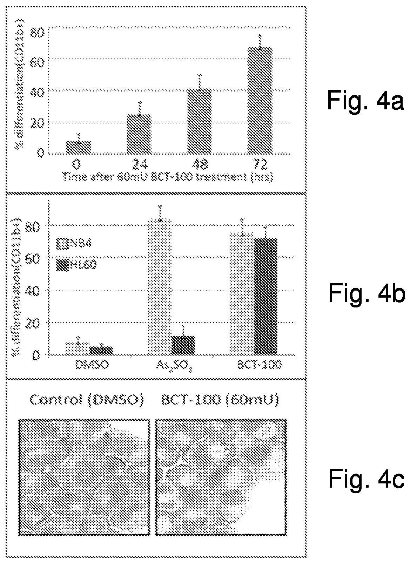

The induction of granulocytic differentiation in NB4 and HL60 cells was studied. Fig. 4a shows that BCT-100 induced granulocytic differentiation in NB4 cells. As revealed by fluorescence-activated cell sorting analysis of CD11b expression shown in Fig. 4b, BCT-100 induced granulocytic differentiation in NB4 and HL60 cells within 96-hour treatment with BCT-100. Fig. 4c shows the granulocytic morphology of NB4 cells which were treated with BCT-100. Specifically, decrease in nuclear to cytoplasmic ratio, appearance of cytoplasmic granules, chromatin condensation and loss of nucleoli were observed.

### Example 6

### Reorganization Of Promyelocytic Leukemia Nuclear Bodies

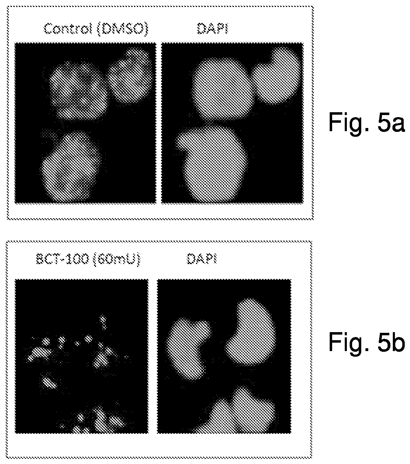

The reorganization of promyelocytic leukemia nuclear bodies in HL60 cells was studied after treatment with BCT-100. Immunostaining with anti-promyelocytic leukemia antibodies revealed a diffusely microspeckled pattern of promyelocytic leukemia in the nuclei of control (DMSO treated) HL60 cells as shown in Fig. 5a. In cells treated with BCT-100, the microspeckled pattern disappeared and the size and brightness of the promyelocytic leukemia bodies returned to normal.

### Example 7

### IC₅₀ of BCT-100 In Various Cancer Cell Lines

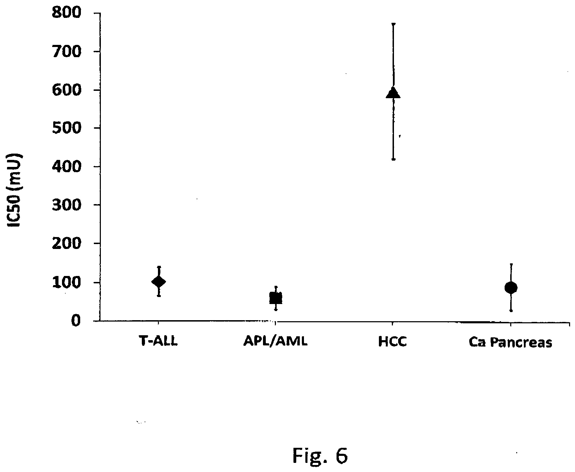

The IC₅₀ values of BCT-100 in various cancer cell lines were investigated. The cancer cell lines being tested were T-cell acute lymphocytic leukemia, acute myeloid leukemia, hepatocellular carcinoma and pancreatic cancer. The results in Fig. 6 showed that BCT-100 is effective in treating T-cell acute lymphocytic leukemia, acute myeloid leukemia, hepatocelluar carcinoma and pancreatic cancer. Further, BCT-100 was found to be at least 6-10 times more potent in treating leukemia and pancreatic cancer than treating hepatocellular carcinoma.

### Example 8

### Effect of BCT-100 On Various Leukemia Cell Lines When Administrated In Combination With Doxorubicin

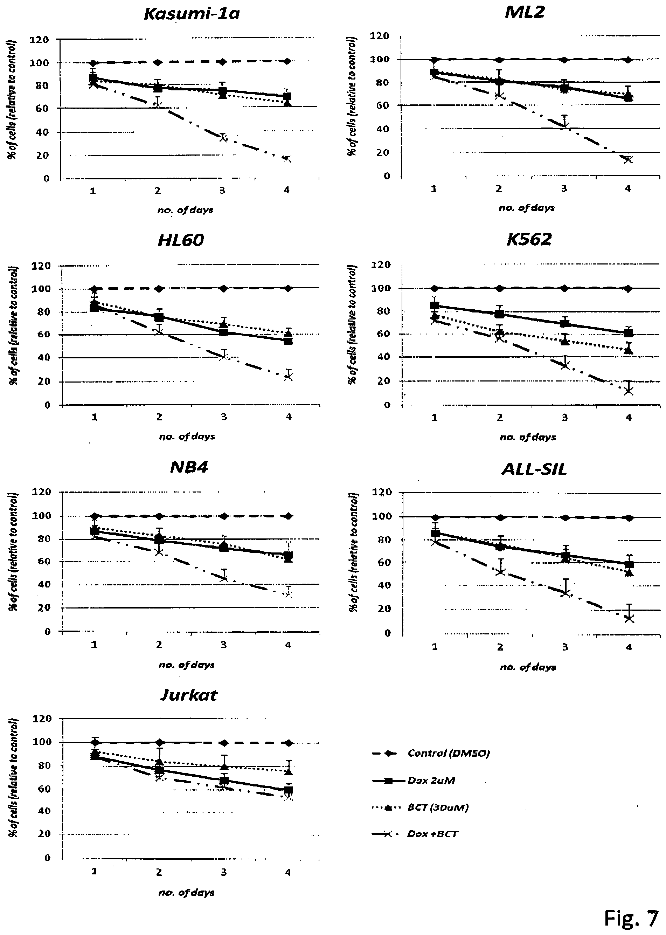

The combination effect of BCT-100 with Doxorubicin on various leukemia cell lines was studied. The leukemia cell lines tested were myeloid leukemia cell lines Kasumi-1a, ML2, HL60, K562, NB4 and lymphocytic leukemia cell lines ALL-SIL and Jurkat. Referring to Figure 7, both BCT-100 and Doxorubicin were found to be effective in treating leukemia, including myeloid and lymphocytic leukemia, when administrated alone respectively. Enhanced therapeutic effect was observed when BCT-100 was administrated in combination with Doxorubicin. The enhancement effect was highly noticeable in all myeloid leukemia cell lines as well as ALL-SIL. The result showed BCT-100 can be administrated with Doxorubicin for the treatment of leukemia, in particular myeloid leukemia and/or lymphocytic leukemia.

### Example 9

### Effect Of BCT-100 On The Liver, Spleen And Sternum Cytology Of HL60-Incoluated NOD/SCIDS Mice

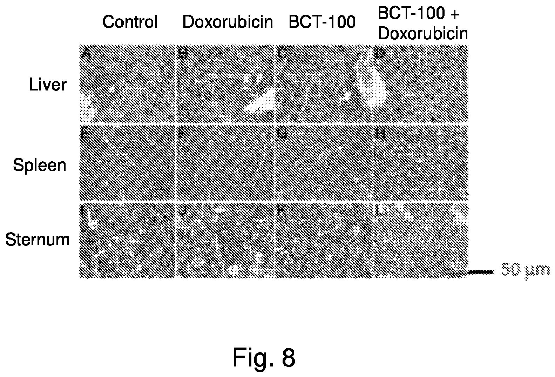

HL60 cells were injected to the tail of NOD/SCIDS mice. Treatment commenced on day 14. The animals were divided into 4 groups. The Doxorubicin group received treatment by doxorubicin, which was administrated at 3mg/kg/day by intraperitoneal injection 3 times weekly for the first week, thereafter once per week for 4 weeks. The BCT-100 group received treatment by BCT-100, which was administrated at 50U/mice by intraperitoneal injection weekly for 4 weeks. The combinational group received treatment of both Doxorubicin and BCT-100, administrated as described above. The control group received intraperitoneal injection of saline. Figure 8 shows the liver, spleen and sternum cytology of the 4 groups of mice after treatment. In the control group, infiltration of leukemia in liver, spleen and sternum was observed. Modest infiltration was observed in the Doxorubicin and BCT-100 group in the liver, spleen and sternum. In the combinational group, note regression of blast from liver and near-normal morphology of sternum was observed.

The result shows that BCT-100 is effective in clearing leukemic blasts in the sternal bone marrow, spleen and liver. BCT-100 can also be administrated in combination with Doxorubicin to enhance the therapeutic effect.

### Example 10

### Enhanced Survival Rate of HL60-incoluated NOD/SCIDS Mice When Treated With BCT-100

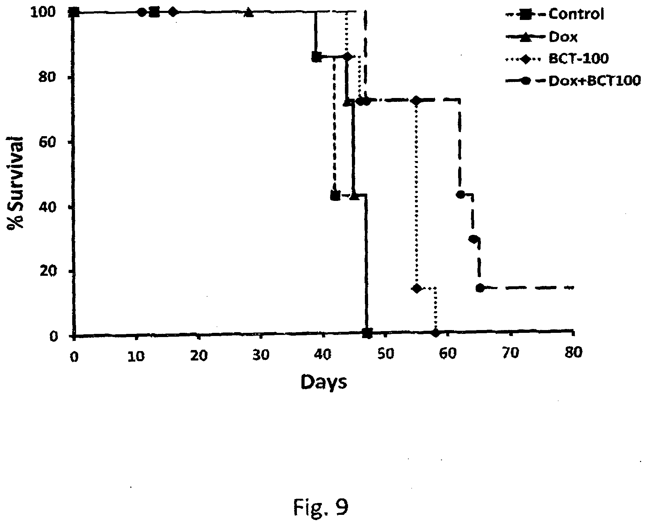

The effect of BCT-100 on the survival rate of HL-60-incoluated NOD/SCIDS mice was investigated. Referring to Figure 9, the survival rate and survival days of mice treated with BCT-100 are increased as compared to the control group, indicating that BCT-100 alone is effective in treating leukemia, e.g., myeloid leukemia. The survival rate and days are further enhanced when BCT-100 is administrated with Doxorubicin. Thus BCT-100 can be administrated with Doxorubicin to treat leukemia, e.g., myeloid leukemia.

### Reference

1. Savoca KV, Davis FF, van Es T, McCoy JR, Palczuk NC. Cancer therapy with chemically modified enzymes. II. The therapeutic effectiveness of arginase, and arginase modified by the covalent attachment of polyethylene glycol, on the taper liver tumor and the L5178Y murine leukemia. Cancer Biochem Biophys 1984;7:261-8
2. L Scott, J Lamb, S Smith and DN Wheatley, Single amino acid (arginine) deprivation: rapid and selective death of cultured transformed and malignant cells, British Journal of Cancer 2000; 83(6), 800-810
3. Storr JM, Burton AF. The effects of arginine deficiency on lymphoma cells. Br J Cancer 1974;30:50-9
4. Denys N Wheatley, Elaine Campbell, Paul BS Lai and Paul NM Cheng. A rational approach to the systemic treatment of cancer involving medium-term depletion of arginine, Gene Therapy Molecular Biology 2005; Vol 9, 33-40
5. Osunkoya BO, Adler WH & Smith RT, Effect of Arginine deficiency on synthesis of DNA and Immunoglobin receptor of Burkitt Lymphoma cells. Nature 1970; 227, 398 - 399
6. H Gong, F Zolzer, G von Recklinghausen, W Havers and L Schweigerer, ADI inhibits proliferation of human leukemia cells more potently than asparaginase by inducing cell cycle arrest and apoptosis. Leukemia 2000; 14, 826-829
7. Cheng PN et al. Remission of hepatocellular carcinoma with arginine depletion induced by systemic release of endogenous hepatic arginase due to transhepatic arterial embolisation, augmented by high-dose insulin: arginase as a potential drug candidate for hepatocellular carcinoma, Cancer Letters 2005; 224, 67-80.
8. Cheng PN et al. Pegylated Recombinant Human Arginase (rhArg-peg5,000mw) Inhibits the In vitro and In vivo Proliferation of Human Hepatocellular Carcinoma through Arginine Depletion, Cancer Research 2007; 67: (1).
9. T L Lam, G K Y Wong, H C Chong, P N M Cheng, S C Choi, S Y Kwok, R T P Poon, D N Wheatley, W H Lo, Y C Leung (2009) Recombinant human arginase inhibits proliferation of human hepatocellular carcinoma by inducing cell cycle arrest, Cancer Letters 2009; 277 (1): 91-100
10. Sam-Mui Tsui; Wai-Man Lam; Tin-Lun Lam; Hiu-Chi Chong; Pui-Kin So; Sui-Yi Kwok; Simon Arnold; Paul Ning-Man Cheng; Denys Wheatley; Wai-Hung Lo and Yun-Chung Leung. Pegylated derivatives of recombinant human arginase (rhArg) for sustained in vivo activity in cancer therapy: preparation, characterization and analysis of their pharmacodynamics in vivo and in vitro action upon hepatocellular carcinoma cell (HCC), Cancer Cell International 2009, 9:9

## Claims

1. A composition comprising arginase for use in the treatment of leukemia in a patient, comprising administrating to the patient a therapeutically effective amount of said composition, wherein said composition is effective at treating lymphocytic and/or myeloid leukemia, wherein said lymphocytic and/or myeloid leukemia is arsenic resistant.

2. The composition for use according to claim 1 wherein said lymphocytic leukemia is acute lymphocytic leukemia or chronic lymphocytic leukemia.

3. The composition for use according to claim 1 wherein said lymphocytic leukemia is T-cell acute lymphocytic leukemia.

4. The composition for use according to claim 1 wherein said myeloid leukemia is acute myeloid leukemia or chronic myeloid leukemia.

5. The composition for use according to claim 1 wherein said arginase is pegylated recombinant human arginase

6. The composition for use according to claim 5 wherein said arginase is pegylated with methoxy-polyethylene glycol succinimidyl propionic acid.

7. The composition for use according to any one of claims 1-6 further comprising administrating a second therapeutic agent, wherein said second therapeutic agent is doxorubicin.

8. The composition for use according to claim 1, wherein said composition is administrated intravenously, intraperitoneally, subcutaneously, or intramuscularly.

9. A composition comprising arginase for use in the treatment of cancer in a patient, comprising administrating to the patient a therapeutically effective amount of said composition, wherein said patient suffers from relapsed or refractory leukemia or lymphoma, wherein said leukemia or lymphoma is arsenic resistant.

10. The composition for use according to claim 9 wherein said arginase is pegylated recombinant human arginase.

11. The composition for use according to claim 10 wherein said arginase is pegylated with methoxy-polyethylene glycol succinimidyl propionic acid.

## Patentansprüche

1. Zusammensetzung, die Arginase umfasst, zur Verwendung in der Leukämiebehandlung bei einem Patienten, umfassend die Verabreichung an den Patienten einer therapeutisch wirksamen Menge besagter Zusammensetzung, wobei besagte Zusammensetzung bei der Behandlung von lymphozytärer und/oder myeloischer Leukämie wirksam ist, wobei besagte lymphozytäre und/oder myeloische Leukämie arsenresistent ist.

2. Zusammensetzung zur Verwendung nach Anspruch 1, wobei besagte lymphozytäre Leukämie akute lymphozytäre Leukämie oder chronische lymphozytäre Leukämie ist.

3. Zusammensetzung zur Verwendung nach Anspruch 1, wobei besagte lymphozytäre Leukämie T-Zell-akute lymphozytäre Leukämie ist.

4. Zusammensetzung zur Verwendung nach Anspruch 1, wobei besagte myeloische Leukämie akute myeloische Leukämie oder chronische myeloische Leukämie ist.

5. Zusammensetzung zur Verwendung nach Anspruch 1, wobei besagte Arginase pegyllierte rekombinante humane Arginase ist.

6. Zusammensetzung zur Verwendung nach Anspruch 5, wobei besagte Arginase mit Methoxy-Polyäthylen-Glykol-Succinimidyl-Propionsäure pegylliert wird.

7. Zusammensetzung zur Verwendung nach einem beliebigen der Ansprüche 1-6, die des Weiteren die Verabreichung eines zweiten therapeutischen Wirkstoffs umfasst, wobei besagter zweite therapeutische Wirkstoff Doxorubicin ist.

8. Zusammensetzung zur Verwendung nach Anspruch 1, wobei besagte Zusammensetzung intravenös, intraperitoneal, subkutan oder intramuskulär verabreicht wird.

9. Zusammensetzung, die Arginase umfasst, zur Verwendung in der Krebsbehandlung bei einem Patienten, umfassend die Verabreichung an den Patienten einer therapeutisch wirksamen Menge besagter Zusammensetzung, wobei besagter Patient unter rezidivierender oder refraktärer Leukämie oder Lymphom leidet, wobei besagte Leukämie oder besagtes Lymphom arsenresistent ist.

10. Zusammensetzung zur Verwendung nach Anspruch 9, wobei besagte Arginase pegyllierte rekombinante humane Arginase ist.

11. Zusammensetzung zur Verwendung nach Anspruch 10, wobei besagte Arginase mit Methoxy-Polyäthylen-Glykol-Succinimidyl-Propionsäure pegylliert wird.

## Revendications

1. Composition comprenant de l'arginase pour une utilisation dans le traitement de la leucémie dans un patient, comprenant l'administration au patient d'une quantité thérapeutiquement efficace de ladite composition, ladite composition étant efficace pour le traitement de la leucémie lymphoïde et/ou myéloïde, ladite leucémie lymphoïde et/ou myéloïde étant résistante à l'arsenic.

2. Composition pour une utilisation selon la revendication 1, dans laquelle ladite leucémie lymphoïde est la leucémie lymphoïde aiguë ou la leucémie lymphoïde chronique.

3. Composition pour une utilisation selon la revendication 1, dans laquelle ladite leucémie lymphoïde est une leucémie lymphoïde aiguë des lymphocytes T.

4. Composition pour une utilisation selon la revendication 1, dans laquelle ladite leucémie myéloïde est la leucémie myéloïde aiguë ou la leucémie myéloïde chronique.

5. Composition pour une utilisation selon la revendication 1, dans laquelle ladite arginase est une arginase humaine recombinante pégylée

6. Composition pour une utilisation selon la revendication 5, dans laquelle ladite arginase est pégylée par de l'acide méthoxy-polyéthylène glycol succinimidyl propionique.

7. Composition pour une utilisation selon l'une quelconque des revendications 1 à 6, comprenant en outre l'administration d'un second agent thérapeutique, dans laquelle ledit second agent thérapeutique est la doxorubicine.

8. Composition pour une utilisation selon la revendication 1, dans laquelle ladite composition est administrée par voie intraveineuse, intrapréritonéale, sous-cutanée ou intramusculaire.

9. Composition comprenant de l'arginase pour une utilisation dans le traitement du cancer dans un patient, comprenant l'administration au patient d'une quantité thérapeutiquement efficace de ladite composition, dans laquelle ledit patient souffre de leucémie ou lymphome en rechute ou réfractaire, ladite leucémie ou ledit lymphome étant résistant(e) à l'arsenic.

10. Composition pour une utilisation selon la revendication 9, dans laquelle ladite arginase est une arginase humaine recombinante pégylée.

11. Composition pour une utilisation selon la revendication 10, dans laquelle ladite arginase est pégylée par de l'acide méthoxy-polyéthylène glycol succinimidyl propionique.
